# EUROPEAN PATENT APPLICATION

(11) **EP 1 101 464 A1**
(43) Date of publication of application: **23.05.2001**
(21) Application number: 00124976.2
(22) Date of filing: 16.11.2000
(51) Int. Cl.: A61F 5/451

(54) **Device for disposing excrement**

(30) Priority: 17.11.1999 JP 32641599
(71) Applicant: Niles Parts Co., Ltd., Tokyo 143-0015 (JP)
(72) Inventor: Oki, Nobuyoshi, C/O Niles Parts Co., Ltd., Tokyo, 143-0015 (JP); Tamura, Shuei, C/O Niles Parts Co., Ltd., Tokyo, 143-0015 (JP); Sakai, Hiroshi, C/O Niles Parts Co., Ltd., Tokyo, 143-0015 (JP); Sato, Kimio, C/O Niles Parts Co., Ltd., Tokyo, 143-0015 (JP)
(74) Representative: Luderschmidt, Schüler & Partner GbR

(57) **Abstract**

A device for disposing excrement is provided which prevents against red in the skin of a sick person or the like wearing a diaper cup. A device for disposing excrement has a diaper cup main body 2 enclosing a pelvic region of a human body, a discharge port 2d to discharge the dirt excreted in the diaper cup main body 2, an anus wash nozzle to eject liquid for washing away the dirt, sensors 13, 14 to detect the dirt excreted in the diaper cup main body 2, and a change diaper 3 removably provided on the diaper cup main body 2. A buffer member 25 is interposed between the diaper cup main body 2 and the change diaper 3.

## Description

### BACKGROUND OF THE INVENTION

### 1. Field of the Invention

The present invention relates to a device for disposing excrement adapted for a sick person disable to walk or aged bedridden in the hospitals and, more particularly, to a device for disposing excrement which is capable of automatically disposing of dirt and washing the places tainted with the dirt a sick or bedridden person excretes.

### 2. Description of the Related Arts

Conventionally, there has been an invention as a device for disposing excrement of this kind disclosed, for example, in Japanese Patent Laid-open No. 364841/1992. This device for disposing excrement has, in a center of a diaper cover, a hole opened to insert through an evacuation pipe to discharge excrement, a pipe to feed warm water, a pipe to feed warm air and a cord connected to a sensor to detect excrement.

Meanwhile, a skin cloth to be attached to the main body has a hole in a surface facing the cup portion. In the left and right of the skin-cloth hole, there are attached respective elongate plate portions firmly fixed with in-line projection buttons so that the skin cloth is attached to the main body through the projection buttons. The main body has, in the cup portion, a washing ejection port, an air-delivery inlet port and an excrement-detecting sensor. The excrement-detecting sensor comprises two feces excrement-detecting sensors arranged extending from around the discharge port to an inside of the discharge port, and three urine excrement-detecting sensors arranged around the discharge port.

However, the related-art device for disposing excrement is delimited in flexibility despite a soft insulative material is employed to meet various body forms. Where wearing a diaper cup for a long time, the sick person or the like would feel pain due to hardness or the like. Thus, there has been a problem of poor in wear feeling.

Meanwhile, there is another problem that, where the sick person or the like wears the diaper cup for a long time, red of skin possibly occur due to a body weight or the like.

The present invention is for solving the problems in the related art, and it is an object of the invention to provide a device for disposing excrement which prevents against occurrence of red in the skin of a sick person or the like wearing a diaper cup and is comfortable to wear.

### SUMMARY OF THE INVENTION

The present invention has been made in order to solve the above-stated problems in the related art and, in a device for disposing excrement having a diaper cup main body for enclosing a pelvic region of a human body, a discharge port for discharging dirt excreted in the diaper cup main body, a sensor for detecting the dirt excreted in the diaper cup main body, and a change diaper removably provided on the diaper cup main body, the excrement disposal apparatus is characterized in that: a buffer member is interposed between a pelvic region of a human body and the diaper cup main body. The buffer member can prevent against red in the skin of a sick person or the like due to his or her body weight wearing the diaper cup main body, and provide an excrement disposal apparatus that does not cause pain even where wearing the diaper cup main body for a long time.

The diaper cup main body preferably has a cavity formed in a surface on a side to be fit on a pelvic region of a human body to put a human body in a non-contact state, and the buffer member being laid in a peripheral material of the cavity of the diaper cup main body beneath a pelvic region of a human body. This makes it possible to retain a location loaded with a pelvic-region body weight by a required minimum buffer member. The change diaper can be thin in the diaper interior entirety, reducing the feeling of physical disorder due to the diaper cup main body or the like having the buffer member worn by a sick person or the like in sleeping.

The buffer member preferably comprises a thick plate member. This makes it possible to increase the cushion effect and prevent against red in the skin.

The buffer member preferably comprises a gel soft material. This makes it possible to dispersedly sustain a body weight of a sick person or the like thereby preventing bedsores. It is further possible to prevent the excreted urine or the like from leaking to an outside of the diaper cup main body due to close contact without gap of the buffer member with the diaper cup main body or change diaper.

The diaper cup main body preferably has a waist-fit seat generally in a plate form provided continuous with an upper end thereof, the buffer member being provided on an upper position of the waste-fit seat. This makes it possible not to immediately contact the comparatively-hard waist-fit seat placed under a waist of a sick person or the like, providing a comfortable device for disposing excrement not giving a feel of pain.

The buffer member is preferably provided projecting at an upper end of the change diaper on a back side of a human body. This makes it possible to cover a step due to a thickness of a change-diaper upper end by the buffer member thereby preventing against red in the skin.

The buffer member is preferably provided inserted in a bag provided in the change diaper, and the change diaper being arranged with the bag for inserting therein the buffer member on an inner side to be fit on a human body. This makes it possible to prevent against wrinkle in a position of the change diaper to be contacted with a pelvic region on a back side of a sick person or the like wearing the diaper cup and hence red caused by pressing such wrinkle on the skin due to a body weight or the like. Also, the buffer member can receive the body weight of the sick person or the like substantially directly, thereby making it possible good for wear feeling on the worn diaper cup.

The bag of the change diaper for inserting therein the buffer member is preferably formed by a waterproof cloth on a side of the cavity. This makes it possible to improve waterproofness of the bag and thereby prevent the diaper cover or sheets at around the change diaper.

The bag of the change diaper for inserting therein the buffer member is preferably arranged with a waterproof sheet in a seam of the waterproof cloth. This makes it possible to prevent water content from intruding through a seam of the bag into the change diaper and hence provide a change diaper giving no uncomfortable wet feeling to a sick person or the like.

The bag of the change diaper for inserting therein the buffer member is preferably provided with a magic tape and has a close cover to close an opening. This makes it possible to facilitate to put the buffer member into and out of the bag. Thus, when the diaper is tainted or moistened, the buffer member can be easily taken out of the bag to wash the change diaper or buffer member.

### BRIEF DESCRIPTION OF THE DRAWINGS

Fig. 1 is a perspective view as a figure showing an embodiment of the present invention;
Fig. 2 is a magnified center lengthwise sectional view of a diaper cup main body as a figure showing the embodiment of the invention;
Fig. 3 is a front view having a part section of the diaper cup main body as a figure showing the embodiment of the invention;
Fig. 4 is a plan view having a part section of the diaper cup main body as a figure showing the embodiment of the invention;
Fig. 5 is a magnified exploded perspective view of concertina joints and waterproof cloth as a figure showing the embodiment of the invention;
Fig. 6 is an explanatory view of an upper cup, lower cup and waist-fit sheet as a figure showing the embodiment of the invention;
Fig. 7 is a magnified perspective view of the upper cup as a figure showing the embodiment of the invention;
Fig. 8 is a schematic view showing an apron of a change diaper as a figure showing the embodiment of the invention;
Fig. 9 is a schematic view showing a setup state of a waterproof dam as a figure showing the embodiment of the invention;
Fig. 10 is a schematic view showing a shape of an outlet port as a figure showing the embodiment of the invention;
Fig. 11 is a waist-region exploded perspective view showing a structure of a hose connection portion of the diaper main body as a figure showing the embodiment of the invention;
Fig. 12 is a schematic view showing a setup state of a waterproof dam as a figure showing the embodiment of the invention;
Fig. 13 is a schematic view showing a setup state of a waterproof dam as a figure showing the embodiment of the invention;
Fig. 14 is a perspective view showing an assembling state as a figure showing the embodiment of the invention;
Fig. 15 is a front view showing a state when assembling the waist-fit seat to the change diaper as a figure showing the embodiment of the invention;
Fig. 16 is a perspective view showing a state when assembling the buffer member to the change diaper as a figure showing the embodiment of the invention;
Fig. 17 is a schematic view showing a setup state of the buffer member as a figure showing the embodiment of the invention;
Fig. 18 is a schematic view showing a setup state of the apron as a figure showing the embodiment of the invention;
Fig. 19 is a schematic view showing a setup state of a rubber string as a figure showing the embodiment of the invention; and
Fig. 20 is a schematic view showing a setup state of a waterproof sheet as a figure showing the embodiment of the invention.

### DETAILED DESCRIPTION OF THE PREFERRED EMBODIMENTS

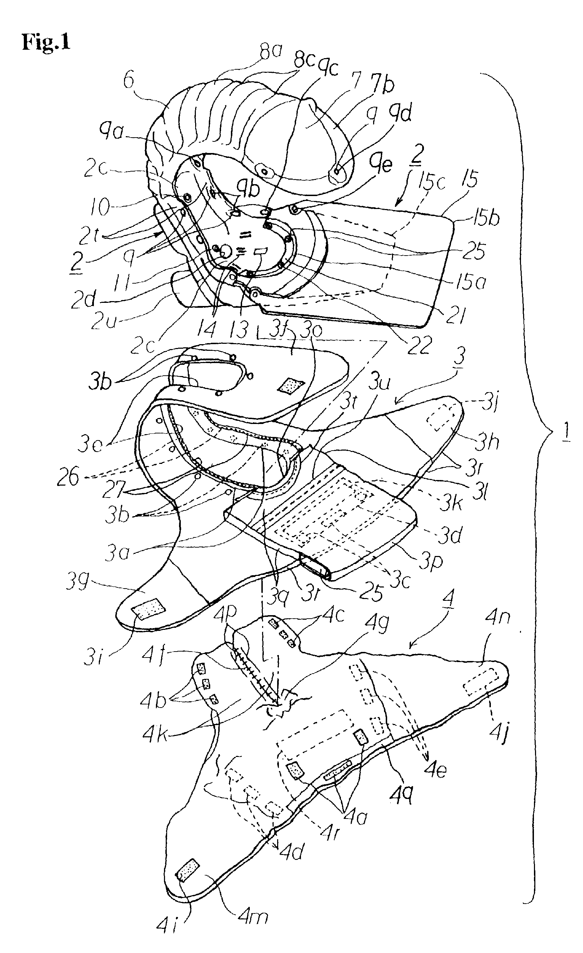

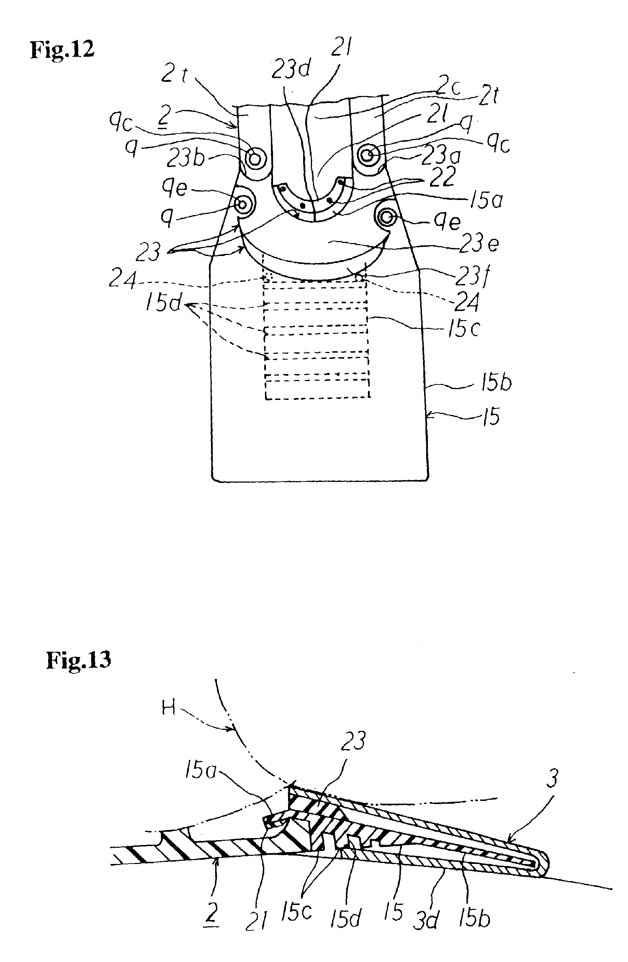

Hereunder, embodiments of the present invention will be explained in detail based on Fig. 1 to Fig. 20.

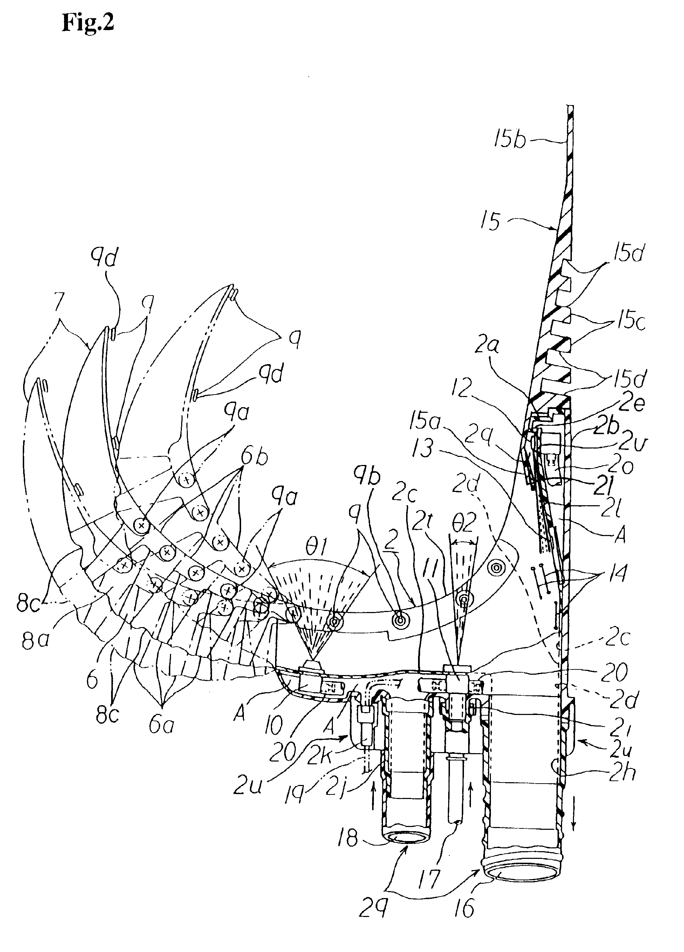

1 is a diaper cup unit as shown in Fig. 1, which comprises a diaper cup main body 2, a change diaper 3 and a diaper cover 4. The diaper main body 2, for enclosing a pelvic region of a human body as shown in Fig. 2, is provided continuous with a multiplicity of concertina joints 6 to have a front cover 7 structured for free bending.

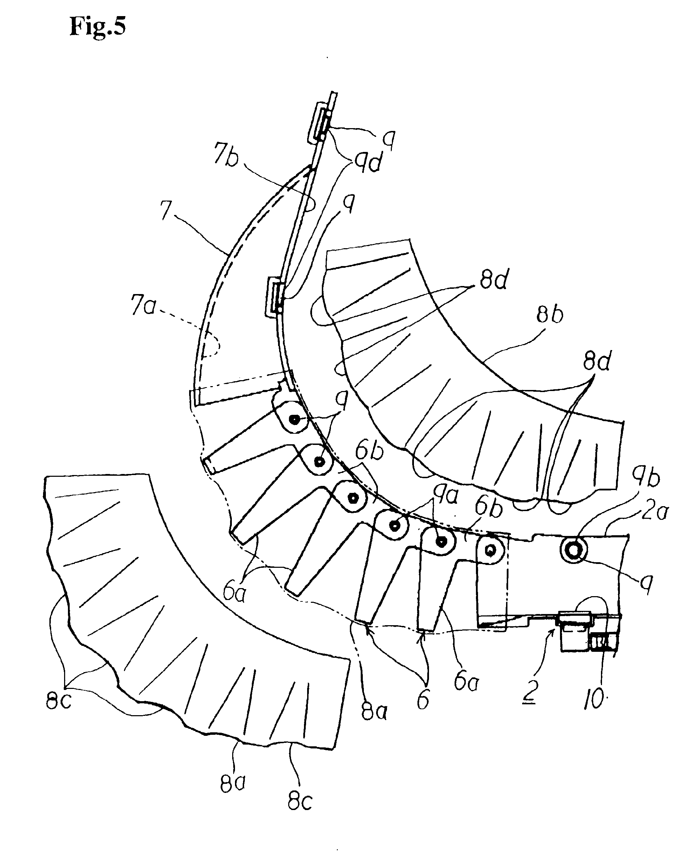

The concertina joints 6 at inner and outer sides are respectively covered with waterproof clothes 8a, 8b as shown in Fig.5, thereby maintaining bend performance of the concertina joints 6 and preventing wash water from leaking. The concertina joints 6 are formed continuous with a cavity 2c, hereinafter described, of the diaper cup main body 2, and comprise a skeleton portion 6a generally in a U-form in section, a connecting portion 6b pivotally supporting each concertina joints 6 for free bend and pivot holes opened to insert hooks 9a in front and back ends of the connecting portion 6b.

The concertina joints 6 are connected continuous at each connecting portions 6b by hooks 9 supporting the change diaper 3 in the pivot holes. Due to this, according to rotate each concertina joints, each concertina joints 6 are allowed to bend and deform as shown by a virtual line in Fig. 2, thus being fitted on a pelvic region different in size by a sick person or the like. Also, the front cover 7 forms, as shown in Fig. 1, Fig. 2 and Fig. 5, a curved recess surface 7a continuing a curved surface of the skeleton portion 6a of the concertina joint 6. The front cover 7 is formed with flanges 7b continuous with the connecting portion 6b of the concertina joint 6 so that the hooks 9d are provided on the flanges 7b.

The waterproof clothes 8a, 8b may be respectively bonded to the whole front and back surfaces, or bonded on front and back surfaces of each of the concertina joints 6. Where the waterproof clothes 8a, 8b, are bonded on each concertina joint 6, six waterproof clothes 8a, 8b for example are provided continuous on the respective front and back surfaces of the six concertina joints 6 while overlapping bonding points. This allows the waterproof clothes 8a, 8b to be bonded in a bendable and deformable state on the front surface generally spherical in form of the concertina joints 6.

Meanwhile, the waterproof clothes 8a, 8b may be formed in one sheet, as shown in Fig. 5. In this case, because the waterproof cloth 8a, 8b is to be bonded on the front and back rounded surfaces of the bend-deformable concertina joints 6, it has spherical areas 8c, 8d formed by pressing a flat strip cloth generally into a spherical form and further a coating with a water preventive agent. The waterproof cloth 8a, 8b is bonded for covering on the concertina portions and skeleton portions 6a of the concertina joints 6 through an adhesive. The waterproof clothes 8a, 8b if formed in one sheet can reduce the number of processes of bonding and dry time of the adhesive.

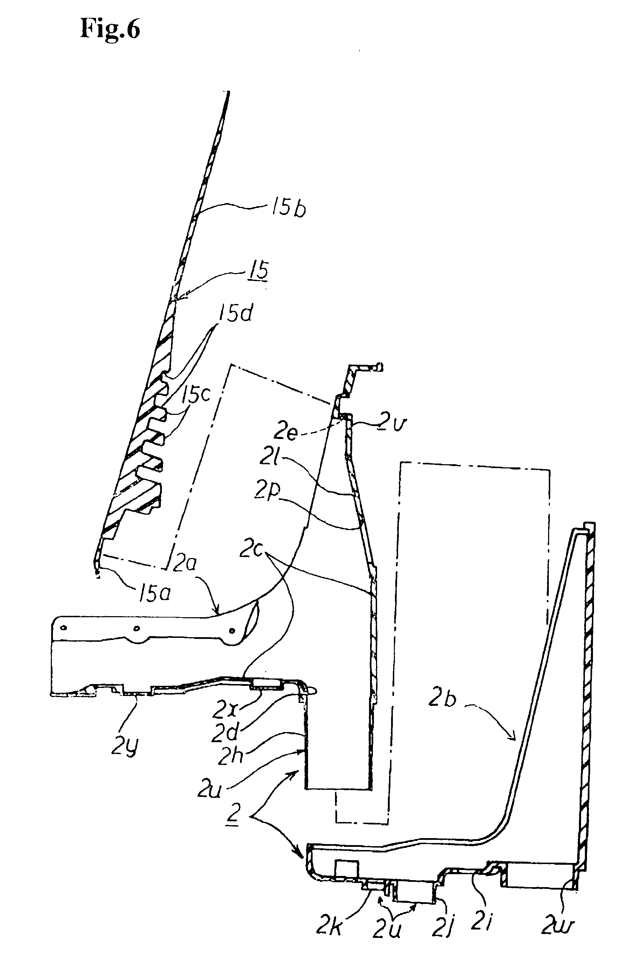

The diaper cup main body 2, as shown in Fig. 6, is bonded together through a space A into one body with an upper cup 2a provided on a side to contact a human body and a lower cup 2b positioned outer when attached to the human body, thus forming a space A at an interior as shown in Fig. 2. The main diaper cup main body 2 is a member connected, into generally u-form, with a front cover 7 to cover a front side of the pelvic region and formed of hard urethane resin and concertina joint 6 and a waist-fit seat 15 to cover a back side of the pelvic region and formed of middle-hard urethane resin for covering an entire pelvic region in order to cover the entire pelvic region.

The diaper cup main body 2 has, as shown in Fig. 1 and Fig. 2, hooks 9b, 9c, a private-parts wash nozzle 10, an anus wash nozzle 11, a cup wash nozzle 12, a feces-detecting sensor 13 and a urine-detecting sensor 14.

The diaper cup main body 2 is connected to an external equipment through use of a dirt-suction hose 16 to suck the wash water and dirt in an inside of the diaper cup unit 1, a wash-water feed hose 17 to feed wash water to the inside of the diaper cup main body 2, a air feed hose 18 to feed warm air to the inside of the diaper cup main body 2, and a sensor coupler 2k to derive electric signals from the feces-detecting sensor 13 and urine-detecting sensor 14.

The upper cup 2a is formed of hard urethane resin or the like generally in a J-form in section. The upper cup 2a is arranged, as shown in Fig. 6, with a discharge port 2d provided at a center on a surface side, a connection cylinder portion 2h continuous to the discharge port 2d and connecting to a dirt-suction hose 16, cavities 2c provided on respective sides of the discharge port 2d, nozzle setup holes 2v formed in the cavities 2c to install cup-wash nozzles 12, a nozzle setup hole 2x to install a anus-wash nozzle 11, a nozzle setup hole 2y to install a private-parts wash nozzle 10, a plurality of the hooks 9b in inner upper ends of side walls on left and right of the cavity 2c, hooks 9c in left and right edges 2t of the cavity 2c, and air-blow ports 2e, 2f, 2g on a side of the waist-fit seat 15.

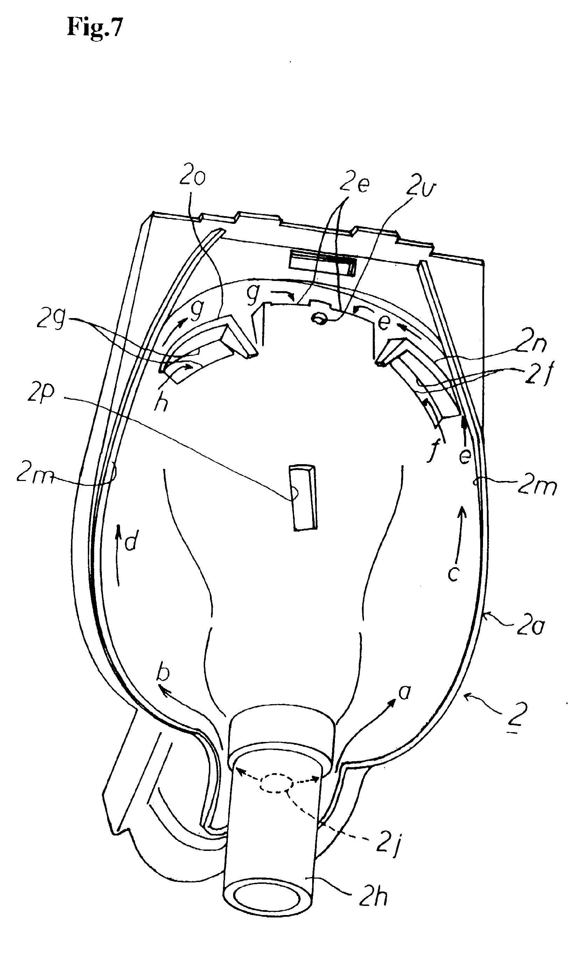

The upper cup 2a is formed, in a joining surface on the backside to the lower cup 2b, with inner walls 2m forming the space A to pass air from the connecting cylinder portion 2j to the air blow ports 2e, 2f, 2g through the air feed hose 18. The air entered in the space A through the connecting cylinder portion 2j is branched to the left and right of the connecting cylinder portion 2h connecting to the dirt suction horses 16 as shown at arrows a, b of Fig. 7, and guided by the inner wall 2m thus flowing toward the air blow ports 2e, 2f, 2g as shown at arrows c, d.

The air at the arrow c, d, at both left and right, is separated into arrows e, f and arrows g, h by a guide wall 2n, 2o generally in an L-form. The air at the arrow e, g flows along the inner wall 2m and passes from the space A to the air hot blow port 2e then going out to a surface of the upper cup 2a. Meanwhile, the air at the arrow f, h is guided on the guide wall 2n, 2o and passes the air blow port 2f, 2g then going out to a surface of the upper cup 2a. Note that 2p in Fig. 7 is a penetration hole for installing a feces-detecting sensor 13.

The guide walls 2n, 2o are walls formed generally in an L-form on center and outer-edge sides of the air blow ports 2f, 2g on the left and right to thereby flow air in equivalent amount and with well balance through three air blow ports 2e, 2f, 2g. The guide walls 2n, 2o are walls to collect air to the left and right air blow ports 2f, 2g thereby providing a function to increase the amount and intensity of air flow through the left and right air blow ports 2f, 2g.

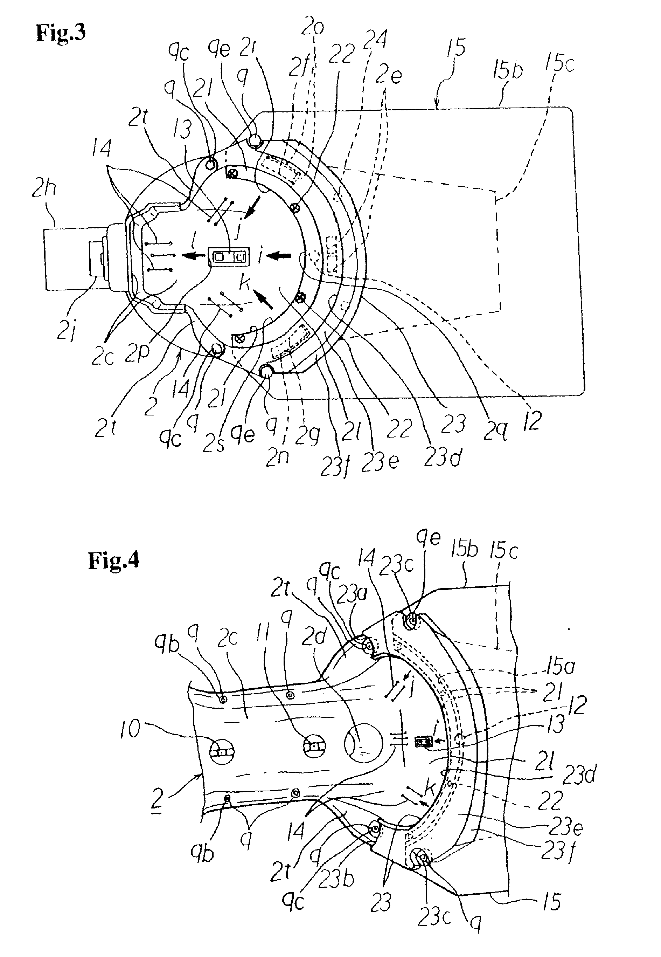

On a surface side of the air blow ports 2e, 2f, 2g, flaps 21 are fixed through screws 22, as shown in Fig. 3, Fig. 4, Fig. 8 and Fig. 9. The flaps 21 are guides to increase the intensity of air flow to the surface of the upper cup 2a through the air blow ports 2e, 2f, 2g to reduce turbulence of air and providing the air with directivity toward a direction of from a bottom of the cavity 2c to the discharge port 2d, thereby flowing excrement and wash water toward the discharge port 2d with smoothness and efficiency.

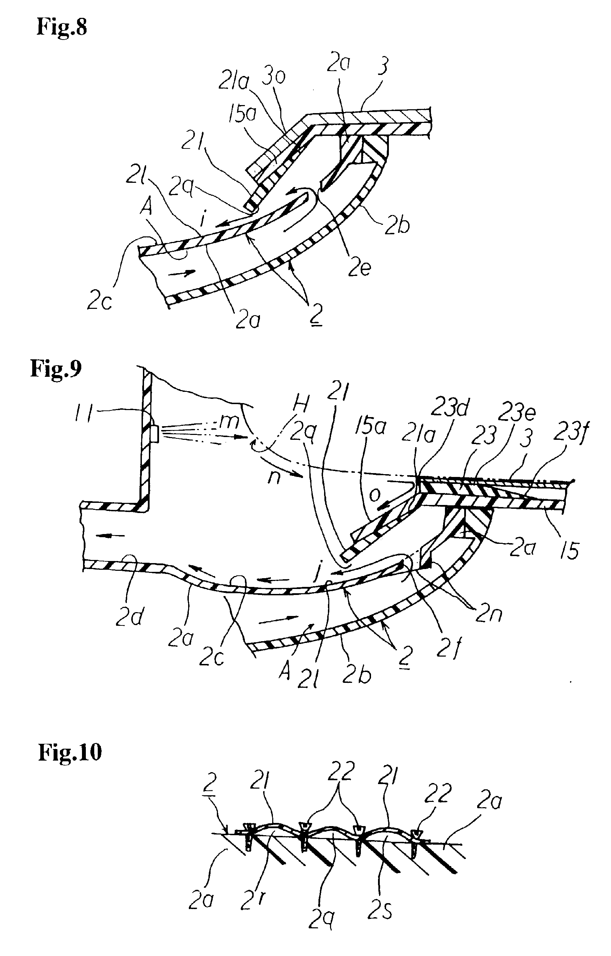

The flap 21 is generally in an arcuate form long in the sideways and formed of a middle hard urethane resin sheet, and has a wedge-formed portion 21 a on a side of the waist-fit seat 15 as shown in Fig. 8 and Fig. 9. The flaps 21 are provided in a manner covering over the air blow ports 2e, 2f, 2g as shown in Fig. 8 and Fig. 9, and arranged at an upper end in a slant surface 21 of the diaper cup main body 2.

The flap 21 is formed with the wedge-formed portion 21a at one end to have the other end to be arranged directed to the slant surface 21 of the diaper cup main body 2, thereby providing air blow port 2q, 2r, 2s as a thickness-reduced port. Also, the flaps 21 can make long the shape and decrease aperture of outlet port 2q, 2r, 2s by fixing each side portions of the three air blow ports 3e, 3f, 3g with four screws 22 on a diaper cup main body 2, for example as shown in Fig. 10. This makes it possible to flow air in a manner immediately along a bottom of the entire slant surface 21 and cavities 2c of the diaper cup main body 2, as arrows i, j shown in Fig. 8 and Fig. 9.

Also, the air blown out of the outlet port 2q, 2r, 2s flows toward the discharge port 2d as shown at arrows i, j, k, l, because the flap 21 is in the arcuate form as shown in Fig. 3 and Fig. 4. Due to this, even where a sick person is in a lying state, wash water and urine are splashed smoothly toward the discharge port 2d side, making possible to prevent against staying of them in the diaper cup main body 2 and erroneous operation for discharging urine by a sick person or the like. Also, feces can be floated and washed, without remainder, out of the cavity 2c of the diaper cup main body 2 by the air from the outlet ports 2q, 2r, 2s and wash water through the anus wash nozzle 11 and cup wash nozzle 12.

Incidentally, the screws 22 in the diaper cup main body 2 use makeup screws made in a same color as the diaper cup main body 2, e.g. with white paint, in order not to provide a sick person with a feel of metal mechanical coolness.

On the flap 21 is provided a support portion 15a generally in an arcuate form formed at an end of the waist-fit seat 15 to prevent the flap 15 from spreading due to the force of airflow and providing reinforcement.

The lower cup 2b of the diaper cup main body 2 is formed of hard urethane resin similar to the upper cup 2a. The lower cup 2b, if joined with an underside of the upper cup 2a, forms a space A to the upper cup 2a and at the same time a diaper main body 2. The lower cup 2b is formed, as shown in Fig. 2, Fig. 6 and Fig. 11, with a hose connecting portion 2u integrally forming a penetration hole 2w in which the connecting cylinder portion 2h of the upper cup 2a is to be fitted, a connecting portion 2i to connect between a wash-water feed hose 17 and a water feed pipe 20, a connecting cylinder portion 2j to connect a air-feed hose 18 to feed air into the space A, and a sensor coupler 2k to connect to a cord 19 connected to a power supply or the like.

The dirt suction hose 16 is connected to a vacuum motor or vacuum pump (not shown) by way of a dirt tank (not shown) and vacuum hose (not shown). The wash-water feed hose 17 is connected to a wash-water ejection adjust valve (not shown). The air feed hose 18 is connected to the vacuum motor or vacuum pump. The sensor coupler 2k is connected to a sequencer (not shown) through a sensor signal line (not shown).

Incidentally, the vacuum motor or vacuum pump is accommodated, together with a filter box, air discharge pipe and motor-cooling air suction pipe, in a housing. Also, the dirt suction hose 16 and the wash-water feed hose 17 are coupled together by a drain valve, and the warm water tank is connected with a water discharge valve.

The space A has, as shown in Fig. 2, a function as a space to lay a water feed pipe 20 connected to the private-parts wash nozzle 10 and anus wash nozzle 11 and cup wash nozzle 12, a function as a space to lay a cord 19 connected to a feces-detecting sensor 13 and urine-detecting sensor 14, and a function as a feed air pipe to feed the air from the air feed hose 18 to the air blow ports 2e, 2f, 2g.

The cavity 2c is a semispherical recess formed in the upper cup 2a, which is formed continuous with a slant surface 21 on the side of the waist-fit seat 15 side as shown in Fig. 2. The cavity 2c is arranged, in its bottom surface, with the private-parts wash nozzle 10, the anus wash nozzle 11, the feces-detecting sensor 13 and the urine-detecting sensor 14. Also, around the cavity 2c and in the front cover 7 are provided in line a plurality of hooks 9 comprising hooks 9a, 9b, 9c, 9d, 9e to attach and detach the change diaper 3.

The private-parts wash nozzle 10 and the anus wash nozzle 11 are nozzles exclusive respectively for individually washing private parts and anus of a sick person wearing the diaper cup unit 1, as shown in Fig. 2. The ejection range θ1 of the private-parts wash nozzle 10 is set wide as compared to the ejection range 02 of the anus wash nozzle 11.

The cup wash nozzle 12 is a nozzle to wash away the dirt excreted by a sick person as shown in Fig. 2, which is arranged on an inner side of the flaps 21 in the vicinity of the air blow port 2e for blowing warm air, to eject wash water toward the feces-detecting sensor 13 side. The cup wash nozzle 12 is fitted in a nozzle setup hole 2v opened in a center nearby the air blow port 2e comprising two, i.e. the left and right, as shown in Fig. 7.

The feces-detecting sensor 13 is a sensor to detect the feces excreted by a sick person or the like. The feces-detecting sensor 13 comprises, for example, one sensor installed at a center location in the slant surface 21, as shown in Fig. 2, Fig. 3 and Fig. 4.

The urine-detecting sensor 14 is a sensor to detect the urine excreted by a sick person or the like. As shown in Fig. 1, Fig. 3 and Fig. 4, sensors comprising, for example, two to three conductor wires are installed, e.g. at three locations, i.e. a location of from the air blow ports 2e, 2f, 2g to a center of the cavity 2c in the vicinity of the lower discharge port 2d and locations on the side walls at left and right thereof, and directed toward the discharge port 2d.

The waist-fit seat 15 is in a plate-formed one on which a sick person or the like rests his or her waist, and formed of middle hard urethane resin having a hardness, e.g. of approximately 70 [Hs]. The waist-fit seat 15 is formed integral with a flat, bendable or deformable plate portion 15b, a support portion 15a formed on a cavity-2c side of the plate portion 15b and a slant portion 15c installed in an outward center.

The waist-fit seat 15 is slant in its inner surface entirety due to possessing the slant portion 15c in the outer center. The slant portion 15c is formed with a multiplicity of cutout grooves 15d in a sideway direction to have a form easy to bend or deform. The waist-fit seat 15 is easy to deform due to the formation of the cutout groove 15d so that a sick person or the like wearing the diaper main body 2 can easily, freely change his or her position. Thus, the sick person or the like is easy to rise from the bed. The waist-fit seat 15 is fixed to the diaper main body 2 through adhesive and further firmly fixed by screws 24.

A waterproof dam 23 generally in an arcuate form is arranged in a location of around the support portion 15a of the waist-fit seat 15 where a coccyx is positioned when the diaper cup main body 2 is worn by a sick person or the like. The waterproof dam 23 comprises a buffer member, for example, of silicone rubber having a hardness of approximately 7 [Hs]. The waterproof dam 23 has both of a role as a buffer member in position that a coccyx of a sick person abuts against and a role as water prevention of preventing wash water or urine from leaking outside the diaper cup main body 2.

In the waterproof dam 23, when the wash water ejected in a direction of arrow m through the anus wash nozzle 11 hits a pelvic region H of a sick person or the like as shown in Fig. 9, the water with the impetus flows in a direction of arrow n along a surface of the pelvic region H to hits against the waterproof dam 23. The wash water hit the waterproof dam 23 then hits an end of the waterproof dam 23 and repelled back in a direction of arrow o to flow through the slant surface 21 of the diaper cup main body 2. Then, the wash water flows together with the air flow in the arrow direction of i, j, k from the exit ports 2q, 2r, 2s in a manner sucked toward the discharge port 2d.

The waterproof dam 23 comprises, as shown in Fig. 4, opposite ends 23a, 23b rested on opposite bank-like edges 2t on both sides of the cavity 2c and arranged matched to a half periphery of the hook 9c, a hook buffer portion 23c covering a half periphery of another hook 9e, a dam portion 23d formed arcuate meeting an outer edge of the slant surface 21 of the diaper cup main body 2, a flat surface portion 23e to receive load of a sick person or the like, and a slant portion 23f formed continuous with the flat surface portion 23e. The waterproof dam 23 is fixed through adhesive on the diaper cup main body 2 and waist-fit seat 15.

The both ends 23a, 23b of the waterproof dam 23 provided at the both edges 2t of the diaper cup main body 2 provides a waterproof effect of eliminating a gap between a pelvic region H of a sick person or the like and the diaper cup main body 2 and preventing wash water or urine from leaking. Also, the both ends 23a, 23b and buffer portion 23c prevent the hooks 9c, 9e provided on the diaper cup main body 2 and waist-fit seat 15 from abutting against a sick person or the like, preventing against red in the skin of the sick person or the like.

Also, the dam portion 23d reflects wash water or urine. Furthermore, it comprises a soft material and hence close contacts the waist-fit seat 15 and change diaper 3, preventing the water content from intruding through between the waist-fit seat 15 and the change diaper 3. This prevents against wetting in the diaper cover 4 or bed sheet. The flat surface portion 23e and slant portion 23f if receiving a body weight of a sick person or the like will not cause red in the skin of the sick person or the like, because the waterproof dam 23 is formed of a soft material.

Next, explanation will be made on the change diaper 3. The change diaper 3 has, as shown in Fig. 1, an elongate hole 3e opened in a center to insert through the hose connecting portion 2u, a side-leak preventive frill 3a provided on left and right of the elongate hole 3e, a magic tape 3c to be joined with a magic tape 4a (registered trademark, hereinafter the same) of the diaper cover 4, a bag 3d that the waist-fit seat 15 is to be inserted, a waist cover 3k for resting thereon the waist of a sick person or the like and a bag 31 that the buffer member 25 is to be inserted.

The change diaper 3 is to be attached by engaging between the hook 9 having the hooks 9a, 9b, 9c, 9d, 9e on the inner side of the diaper cup main body 2 and the hook 3b provided around the elongate hole 3e, thereby being wound around the pelvic region H of a sick person or the like. The change diaper 3 is provided around the cavity 2c of the diaper cup main body 2, and comprises a wrap portion 3f provided around the cavity 2c of the diaper cup main body 2 to cover the pelvic region H of a sick person or the like, and a tightening strip portions 3g, 3h for turning the waist of the sick person or the like. The change diaper 3 is formed, for example, of a nylon cloth material generally in a T form in its entirety.

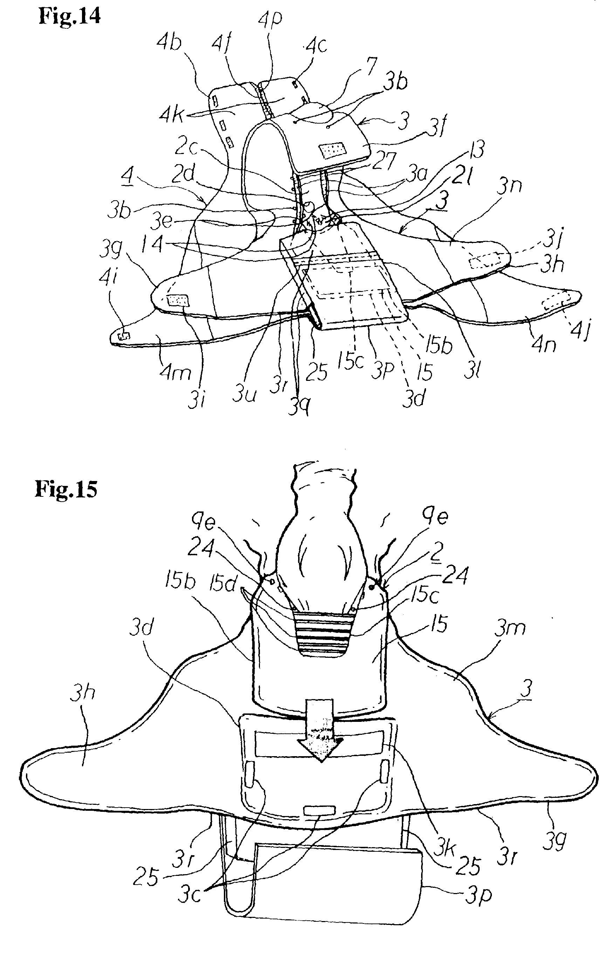

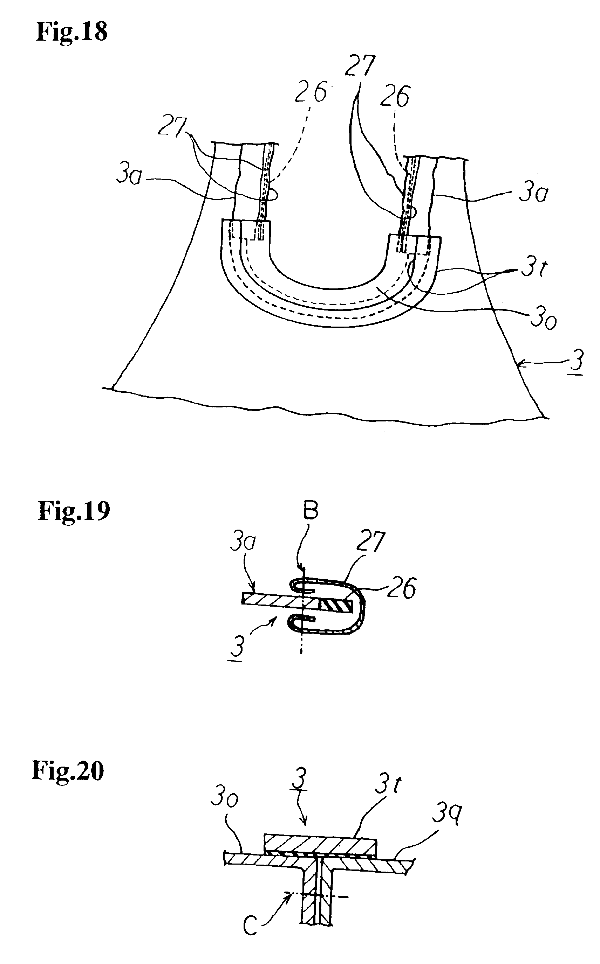

The side-leak preventive frills 3a are provided on the both sides of the elongate hole 3e on the inner side of the change diaper 3 in a manner covering a head of the hook 3b, as shown in Fig. 1 and Fig. 14. The side-leak preventive frill 3a is formed, for example, of a waterproof-treated nylon cloth and sewed with corrugation for expansion and contraction. The left and right side-leak preventive frills 3a have, at ends, respective waterproof clothes 27 internally provided with a rubber string 26, as shown in Fig. 18.

The rubber string 26 is sewed in an end of the side-leak preventive frill 3a, as shown in Fig. 18 and Fig. 19. The rubber string 26 is covered by waterproof cloth 27, and sewed to the side-leak preventive frill 3a by a waterproof thread B with the waterproof cloth 27 bent at its both ends. This prevents against wetting in an outer side of the change diaper 3 or diaper cover 4 due to convey through the rubber string 26 having a nature of absorbing water content.

The hook 3b is firmly fixed, for example, in a hole opened around the elongate hole 3e in the change diaper 3, by press-fitting the members on the front and back sides. The hook 3b is provided in a hidden state at the rear of the side-leak preventive frill 3a on the inner side of the change diaper 3, as shown in Fig. 1 and Fig. 14. The hook 3b fits with the hooks 9b, 9c, 9e provided around the cavity 2c of the diaper cup main body 2, the hook 9a provided at the edge of the concertina joints 6 continuing to the cavity 2c, and the hook 9d provided at the flange 7b of the front cover 7 continuing to the concertina joints 6, thereby fixing the change diaper 3. The magic tapes 3c, 3i, 3j can be adapted in length by adjusting positions of combination even where there is difference in the size of pelvic region H of a person to wear the change diaper 3.

The tightening strip portions 3g, 3h is attached with magic tapes 3i, 3j in the respective left and right corresponding positions as shown in Fig. 1 and Fig. 14, thus serving as a belt to wear the diaper cup main body 2 on a pelvic region H of a sick person or the like. Also, the change diaper 3 comprises a surface cloth 3m, a lining cloth 3n, a mesh cloth (not shown) easy to absorb water that is interposed between the surface cloth 3m and the lining cloth 3n. The mesh cloth comprises a nylon cloth improved in absorbability, for example, by increasing the in-cloth space. The surface cloth 3m and the lining cloth 3n are sewed with dense cloth fiber as compared to the mesh.

Incidentally, in the change diaper 3, where a waterproof dam 23 is not provided in the diaper cup unit 1, an apron 3o is provided formed of a waterproof cloth in an edge on a bag 31 side of the elongate hole 3e. The apron 3o is provided extending from the support portion 15a of the waist-fit seat 15 into the diaper cup main body 2, as shown in Fig. 8, Fig. 16 and Fig. 18. This prevents wash water or urine from leaking through between the waist-fit seat 15 in a peripheral position of the apron 3o and the change diaper 3.

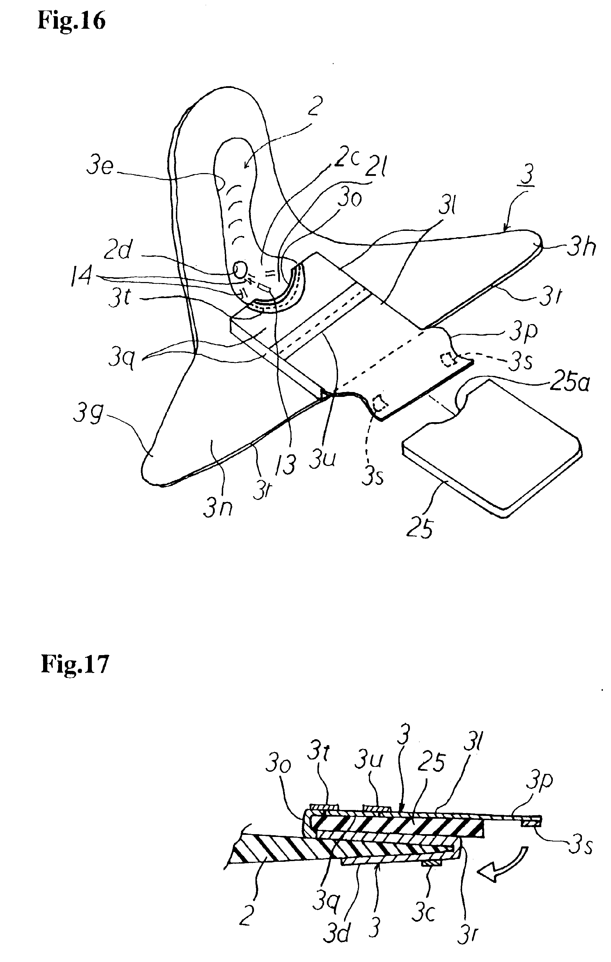

As shown in Fig. 16, Fig. 18 and Fig. 20, water preventive sheets 3t, 3u are bonded on a seam C between the apron 3o and the bag 31 and a seam between the waterproof cloth 3q and the bag 31. This can prevent water content from leaking through the seam of the apron 3o and bag 31 or a seam of the waterproof cloth 3q and bag 31.

The change diaper 3 on a backside is formed with a bag 3d to insert therein the plate-like portion 15b of the waist-fit seat 15, as shown in Fig. 15. By inserting the plate-like portion 15b of the seat, the change diaper 3 is prevented against rumples due to the rigidity of the plate-like portion 15b. Thus, the skin of a sick person or the like will be contacted with a stretched state of the change diaper 3 without rumples, preventing against red in the sick person's skin. The bag 31 for inserting the buffer member 25 is formed on a surface side of the change diaper 3 to be contacted with a pelvic region H, as shown in Fig. 1, Fig. 14, Fig. 16 and Fig. 17.

The bag 31 has a close cover 3p to cover an insertion port for inserting the damper member 25. The buffer member 25 put in the bag 31 can be fixed by covering the close cover 3p and joining the magic tape 3s to the magic tape 3c on the surface side, as shown in Fig. 16. The side surface and elongate 3e side of the bag 31 comprises a waterproof cloth 3q.

The buffer member 25 comprises a gel sheet that can receive dispersedly a body weight of a sick person or the like. This prevents bedsores of the sick person or the like. The buffer member 25 comprises, for example, a soft, thick plate called Actionpad (registered trademark, hereinafter the same). The Actionpad will not have a reaction force upon undergoing a weight, which is free from adhesion at bottom and has a pressure-dispersion effect. The Actionpad is soft and ready to deform upon undergoing load but excellent in restoration force. Also, the Actionpad has a nature of not filled with temperature having an effect of sultry-prevention effect as well as an effect of not causing red in the skin.

The buffer member 25 and bag 31 at one end close to the cavity 2c is formed with a cutout portion 25a in an arcuate form matched to the shape of the dam portion 23d of the waterproof dam 23, as shown in Fig. 16. The other end of the buffer member 25 is formed long to extend from the upper end 3r, as shown in Fig. 1, Fig. 14 and Fig. 16. This can prevents the upper end 3r of the change diaper 3 from pressing against a sick person or the like and causing red in the skin.

Next, explanations will be made on the diaper cover 4.

The diaper cover 4 has, as shown in Fig. 1, magic tapes 4a, 4b, 4c, 4d, 4e, 4i, 4j, a fastener 4f, a hose-unit pass hole 4g and a hose-unit enclosure portion (not shown).

The diaper cover 4 is generally in a T-form alike the change diaper 3, which is a cloth-make member comprising a pelvic-region enclosure portion 4k to cover the enclosure portion 3f, a tightening strip portion 3g, and wrap portions 4m, 4n generally in a T-form to wrap 3h. The diaper cover 4 has a cut portion 4p extending from the hose-unit enclosure portion to the hose-unit pass hole 4g and separate the diaper cover 4 to its outer side. The fastener 4f is arranged in the cut portion 4p, enabling for developing.

The diaper cover 4 and the change diaper 3 are attached together by joining the magic tape 4a to the magic tape 3c on the change diaper 3 side. The diaper cover 4 itself is fixed on a human body by joining the magic tapes 4a, 4b, 4c, 4e, 4i, 4j to a French-pile-make cloth of the diaper cover 4. The fastener 4f is provided so that the fastener 4f can be opened to take out only the diaper cover 4 even when the diaper cup main body 2 is connected with the hose connecting portion 2u.

The pelvic-region enclosure portion 4k is made for free development by separating from the hose-unit enclosure portion below the hose-unit pass hole 4g to an upper end and sewing a fastener 4f thereon. The hose-unit pass hole 4g is an elongate hole coincident with the hose connecting portion 2u and made long along a centerline of the diaper cover 4.

The hose-unit enclosure portion is in a cylindrical form provided continuous with the hose-unit pass hole to cover and hide the hose connecting portion 2u. The hose-unit enclosure portion has a fastener 4f arranged longitudinally in a front center as shown in Fig. 1 and Fig. 14 so that the cylindrical hose-unit enclosure portion is to be developed by opening the fastener 4f.

On a backside upper side of the diaper cover 4, an expansion-and-contraction rubber 4q is internally provided as shown in Fig. 1 so that the diaper cover 4 in area around the upper end fits a waist region of a human body. Also, a waist fit 4r is arranged as a cushion member in a generally center portion of the diaper cover 4.

With the diaper cup main body 2, change diaper 3 and diaper cover 4 explained above, the change diaper 3 and diaper cup main body 2 is fastened by the hooks 9a, 9b, 9c, 9d, 9e, 3b. Next, the change diaper 3 and the diaper cover 4 are fastened by the magic tapes 3c, 3i, 3j, 4a, 4b, 4c, 4e, 4i, 4j. Thus, a diaper cup unit 1 is completed as shown in Fig. 14.

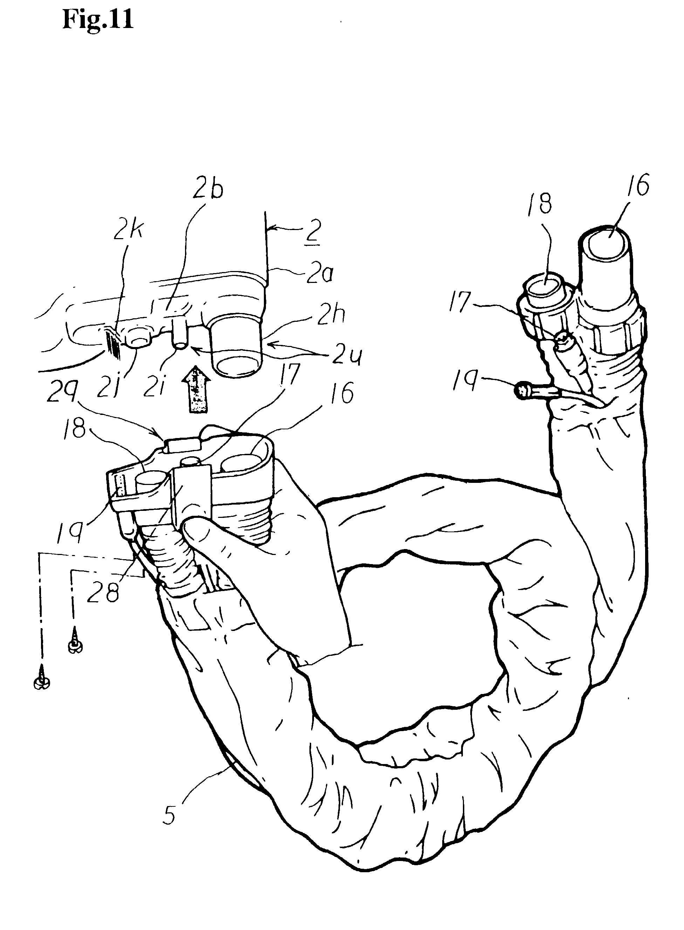

In the diaper cup unit 1, the hose unit 29 is connected to the hose connecting portion 2u as shown in Fig. 11. The hose unit 29 is to connect the diaper cup unit 1 to external equipment, which has the dirt absorbing hose 16, wash-water feed hose 17, air feed hose 18 and sensor coupler 2k in one body and collectively supports them. The hose unit 29 is free to attach to and detach from the hose connecting portion 2u positioned in the underside of the diaper cup main body 2. A hold piece 28 is fixed to hardware (not shown) on a side of the diaper cup main body 2. 5 is a hose cover to be provided continuous with the hose-unit enclosure portion (not shown) of the diaper cover 4 in a manner of overlapping same.

## Claims

1. In a device for disposing excrement having a diaper cup main body for enclosing a pelvic region of a human body, a discharge port for discharging dirt excreted in the diaper cup main body, a sensor for detecting the dirt excreted in the diaper cup main body, and a change diaper removably provided on the diaper cup main body, the device for disposing excrement is characterized in that:
a buffer member is interposed between a pelvic region of a human body and said diaper cup main body.

2. A device for disposing excrement as claimed in claim 1, wherein said diaper cup main body has a cavity formed in a surface on a side to be fit on a pelvic region of a human body to put a human body in a non-contact state, and
said buffer member being laid in a peripheral material of said cavity of said diaper cup main body beneath a pelvic region of a human body.

3. A device for disposing excrement as claimed in claim 1 or 2, wherein said buffer member comprises a thick plate member.

4. A device for disposing excrement as claimed in claim 1, 2 or 3, wherein said buffer member comprises a gel soft material.

5. A device for disposing excrement as claimed in claim 1, 2, 3 or 4, wherein said diaper cup main body has a waist-fit seat generally in a plate form provided continuous with an upper end thereof,
said buffer member being provided on an upper position of said waste-fit seat.

6. A device for disposing excrement as claimed in claim 1, 2, 3, 4 or 5, wherein said buffer member is provided projecting at an upper end of said change diaper on a back side of a human body.

7. A device for disposing excrement as claimed in claim 7, wherein said buffer member is provided inserted in a bag provided in said change diaper, and
said change diaper being arranged with said bag for inserting therein said buffer member on an inner side to be fit on a human body.

8. A device for disposing excrement as claimed in claim 7, wherein said bag of said change diaper for inserting therein said buffer member is formed by a waterproof cloth on a side of said cavity.

9. A device for disposing excrement as claimed in claim 8, wherein said bag of said change diaper for inserting therein said buffer member is arranged with a waterproof sheet in a seam of said waterproof cloth.

10. A device for disposing excrement as claimed in claim 7, 8 or 9, wherein said bag of said change diaper for inserting therein said buffer member is provided with a magic tape and has a close cover to close an opening.
